# EUROPEAN PATENT APPLICATION

(11) **EP 4 155 380 A1**
(43) Date of publication of application: **29.03.2023**
(21) Application number: 21808575.1
(22) Date of filing: 07.05.2021
(51) Int. Cl.: C12M 1/00, C12M 1/26, G01N 1/00, G01N 1/10, G01N 33/48, G01N 33/50

(54) **KIT AND METHOD FOR COLLECTING BIOLOGICAL SUBSTANCE**

(30) Priority: 20.05.2020 JP 2020088191; 17.07.2020 JP 2020122748
(71) Applicant: Universal Bio Research Co., Ltd., Matsudo-shi Chiba 271-0064 (JP)
(72) Inventor: TAJIMA Hideji, Matsudo-shi, Chiba 271-0064 (JP)
(74) Representative: J A Kemp LLP
(86) International application number: PCT/JP2021/017447
(87) International publication number: WO 2021/235236

(57) **Abstract**

The collection kit 1 according to the present invention comprises a collection tool 10 for collecting saliva, a container body for housing the collection tool 10 and a preservation solution for saliva, and a cap for closing the container body. The collection tool 10 comprises a saliva absorbent 12 for collecting saliva, a housing body 14 for housing the saliva absorbent 12, and a pressing body 16 for pressing a collection body 12 and forming a suction space S that is for sucking the saliva and the preservation solution from the outside of the container body 22.

## Description

### TECHNICAL FIELD

The present invention relates to a kit and a method for collecting a biological substance, and more particularly, to a kit and a method for collecting a biological substance so as to collect a pathogen such as a virus contained in the biological substance such as saliva.

### BACKGROUND ART

PCR method utilizing the polymerase chain reaction (PCR), which has become popular with the development of genetic engineering, has been employed to detect a pathogen such as a virus. A biological substance such as saliva containing a pathogen is collected using a commercially available collection kit to extract nucleic acids (DNAs or RNAs) from the biological substance, and the nucleic acids are amplified by PCR method to detect the pathogen based on the amplified nucleic acids.

The world has been exposed to the novel coronavirus (COVID-19) pandemic since the end of 2019. PCR method has been used to detect COVID-19. At present, in order to collect COVID-19 from the human body, a swab is inserted into the nose to collect nasopharyngeal mucus to extract COVID-19 from the collected mucus, and the nucleic acids of COVID-19 are amplified by PCR method, which involves a lot of manual work, to detect COVID-19 based on the amplified nucleic acids. Non-patent literature 1 describes the superiority of the technique that collects COVID-19 from saliva compared to the technique that collects COVID-19 from nasopharyngeal mucus in detecting COVID-19 using PCR method.

In the meantime, a collection kit (biological sample collection device) for collecting saliva, etc. is proposed in, for example, Patent literature 1. The biological sample collection device of Patent literature 1 collects saliva by using an absorbent 12 secured to the tip of a collecting body 10.

### PRIOR ART LITERATURE

### Non-patent literature

Non-patent literature 1: Anne L. Wyllie, et. al, "Saliva is more sensitive for SARS-CoV-2 detection in COVID-19 patients than nasopharyngeal swabs," medRxiv, April 16, 2020

### Patent literature

Patent literature 1: Japanese Unexamined Patent Application Publication No. 2016-170033

### SUMMARY OF INVENTION

### Problem to be solved by invention

When detecting COVID-19 using PCR method, there is a limit to the number of detection that can be carried out and there is a risk of contamination because not all processes are automated and some processes are performed manually. Moreover, the biological sample collection device described in Patent literature 1 collects saliva with an absorbent 12 and squeezes the absorbent 12 with a roller on a table 7 to analyze the obtained saliva. Therefore, in the biological sample collection device described in Patent literature 1, manual work of transferring the saliva obtained by squeezing with the roller to a detector is required, which limits the number of detection that can be carried out and which may cause contamination.

Therefore, the present invention aims at providing a kit and a method for collecting a biological substance, which can efficiently provide a biological substance such as saliva to a detector such as a PCR device.

### Means for solving the problem

Each aspect of the present invention is as follows.
[Aspect 1] A kit for collecting a biological substance, the kit comprising a collection tool for collecting the biological substance, a container body for housing the collection tool and a preservation solution for preserving the biological substance, and a cap member for sealing the container body, wherein the collection tool comprises a collecting body for collecting the biological substance, a housing for housing the collecting body, and a suction space for suctioning the biological substance and the preservation solution from the outside of the container body.
[Aspect 2] The kit for collecting a biological substance according to Aspect 1, wherein the cap member comprises a cap-side insertion opening connecting to the suction space, and a seal for sealing the cap-side insertion opening.
[Aspect 3] The kit for collecting a biological substance according to Aspect 2, wherein the seal is puncturable from the outside.
[Aspect 4] The kit for collecting a biological substance according to any one of Aspects 1 to 3, wherein:
   the collecting body is formed of a stretchable material that can absorb the biological substance and the preservation solution; and
   in an uncompressed state where the collecting body placed in the container body is not compressed, one end of the collection tool sticks out from the container body.
[Aspect 5] The kit for collecting a biological substance according to Aspect 4, wherein, in a compressed state where the collecting body placed in the container body is compressed, said one end of the collection tool is housed in the container body.
[Aspect 6] The kit for collecting a biological substance according to Aspect 5, wherein the container body in the compressed state is sealed with the cap member.
[Aspect 7] The kit for collecting a biological substance according to any one of Aspects 1 to 6, wherein the housing has one or a plurality of openings.
[Aspect 8] The kit for collecting a biological substance according to any one of Aspects 1 to 7, wherein the housing is an elongated basket body.
[Aspect 9] The kit for collecting a biological substance according to any one of Aspects 1 to 8, wherein the collection tool comprises a pressing body that presses the collecting body toward the housing.
[Aspect 10] The kit for collecting a biological substance according to Aspect 9, wherein the pressing body forms the suction space.
[Aspect 11] The kit for collecting a biological substance according to either one of Aspects 10 and 11, wherein the pressing body is tubular.
[Aspect 12] The kit for collecting a biological substance according to any one of Aspects 9 to 11, wherein the collection tool comprises a housing head that holds the pressing body in a slidable manner with respect to the collecting body.
[Aspect 13] The kit for collecting a biological substance according to Aspect 12, wherein the housing head is secured to the housing.
[Aspect 14] The kit for collecting a biological substance according to either one of Aspects 12 and 13, wherein the housing head comprises one or a plurality of protruding portions that make contact with the pressing body.
[Aspect 15] A kit for collecting a biological substance, the kit comprising a collection tool for collecting the biological substance, a container body for housing the collection tool and a preservation solution for preserving the biological substance, and a cap member for sealing the container body, wherein the collection tool comprises a collecting body for collecting the biological substance, and a support for holding the collecting body and forming a suction space for suctioning the biological substance and the preservation solution from the outside of the container body.
[Aspect 16] The kit for collecting a biological substance according to Aspect 15, wherein the cap member comprises a cap-side insertion opening connecting to the suction space, and a seal for sealing the cap-side insertion opening.
[Aspect 17] The kit for collecting a biological substance according to Aspect 16, wherein the seal is puncturable from the outside.
[Aspect 18] The kit for collecting a biological substance according to any one of Aspects 15 to 17, wherein:
   the collecting body is formed of a stretchable material that can absorb liquid; and
   in an uncompressed state where the collecting body placed in the container body is not compressed, one end of the support sticks out from the container body.
[Aspect 19] The kit for collecting a biological substance according to Aspect 18, wherein, in a compressed state where the collecting body placed in the container body is compressed, said one end of the support is housed in the container body.
[Aspect 20] The kit for collecting a biological substance according to Aspect 19, wherein the container body in the compressed state is sealed with the cap member.
[Aspect 21] The kit for collecting a biological substance according to Aspect 20, wherein the preservation solution is contained in the container body in a liquid amount such that, in the compressed state, the liquid level of the preservation solution is located in the suction space.
[Aspect 22] The kit for collecting a biological substance according to any one of Aspects 15 to 21, wherein the support comprises a support-side insertion opening for suctioning the biological substance and the preservation solution from the outside.
[Aspect 23] The kit for collecting a biological substance according to any one of Aspects 15 to 22, wherein the support comprises one or a plurality of side openings opening on the inner surface of the container body.
[Aspect 24] The kit for collecting a biological substance according to any one of Aspects 15 to 23, wherein the support comprises a holding section for holding the collecting body.
[Aspect 25] The kit for collecting a biological substance according to Aspect 24, wherein the holding section comprises holes at its upper and lower ends, each of which allowing communication of the preservation solution and the biological substance.
[Aspect 26] The kit for collecting a biological substance according to any one of Aspects 1 to 25, wherein the container body comprises a label, and the label has at least one of a bar code, a QR code (registered trademark), and a blank name space on which information of the subject can be written.
[Aspect 27] The kit for collecting a biological substance according to any one of Aspects 1 to 26, wherein the biological substance is saliva.
[Aspect 28] A method for collecting a biological substance using the kit for collecting a biological substance according to any one of Aspects 1 to 27, the method comprising the steps of:
   collecting the biological substance into the collection tool;
   housing the collection tool in the container body;
   compressing the collecting body by pressing one end of the pressing body to put the collecting body into the compressed state;
   expanding the collecting body by releasing the pressure at one end of the pressing body to put the collecting body into the uncompressed state; and
   sealing the container body with the cap member, with the collecting body in the compressed state.
[Aspect 29] The method for collecting a biological substance according to Aspect 28, wherein the step of compressing the collection tool and the step of expanding the collection tool are repeated alternately.
[Aspect 30] A testing device for testing a biological substance collected using the kit for collecting a biological substance according to any one of Aspects 1 to 27, the device comprising a dispenser for suctioning the biological substance and the preservation solution from the suction space.
[Aspect 31] A testing device for testing a biological substance collected using the kit for collecting a biological substance according to either one of Aspects 3 and 17, the device comprising:
   a puncturing tool for puncturing the puncturable seal; and
   a dispenser that is inserted into the suction space via the cap-side insertion opening to suction the biological substance and the preservation solution from the suction space.
[Aspect 32] The testing device according to either one of Aspects 30 and 31, wherein the testing device extracts nucleic acids from the biological substance, amplifies the extracted nucleic acids, and tests the amplified nucleic acids.

### EFFECTS OF INVENTION

A kit and a method for collecting a biological substance according to the present invention can efficiently provide the collected biological substance to a testing device.

### BRIEF DESCRIPTION OF DRAWINGS

[Figure 1] A perspective view showing a collection kit according to a first embodiment of the present invention.
[Figure 2] An exploded side view of the collection tool according to the first embodiment of the present invention.
[Figure 3] A side view of a saliva collecting body and a housing shown in Figure 2.
[Figure 4] A perspective view showing the assembled state of the collection tool shown in Figure 2.
[Figure 5] A side view showing the state before the collection tool with the collected saliva is housed in the container body.
[Figure 6] A side view showing the state after the collection tool with the collected saliva is housed in the container body.
[Figure 7] A perspective view showing the collection kit after the collection of saliva.
[Figure 8] A perspective view showing the process of suctioning saliva from the collection kit shown in Figure 7.
[Figure 9] A perspective view showing a collection kit according to a second embodiment of the present invention.
[Figure 10] An exploded perspective view of the collection tool shown in Figure 9.
[Figure 11] An exploded perspective view of the collection kit shown in Figure 9.
[Figure 12] A schematic diagram showing a process of dissolving saliva using the collection kit shown in Figure 9.
[Figure 13] A schematic diagram showing a process of suction from the collection kit shown in Figure 9.

### MODE FOR CARRYING OUT INVENTION

Embodiments concerning a kit and a method for collecting a biological substance (e.g., saliva) of the present invention will each be described with reference to the drawings. In each drawing, identical parts are denoted by the same reference numerals and the descriptions thereof are omitted as appropriate. In each drawing, the relative size and/or arrangement of each part is, in principle, accurately illustrated, but the present invention is not limited thereto. In addition, according to the present invention, the biological substance to be collected is not limited to saliva, but can include any biological substance such as body fluid collected from a living body.

### [First embodiment]

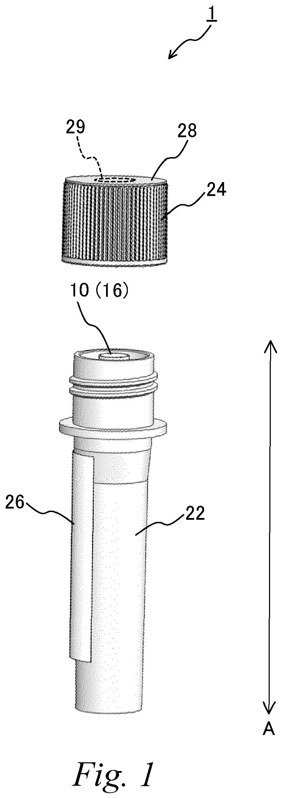

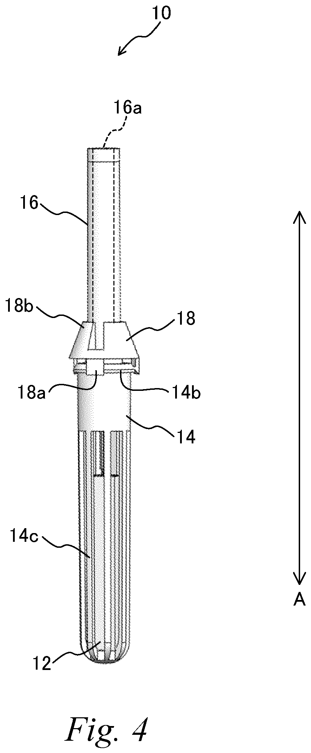

Configuration of a kit 1 for collecting a biological substance according to a first embodiment of the present invention will be described with reference to Figures 1-4. As shown in Figure 1, the collection kit 1 comprises a saliva collection tool 10, a container body 22 for housing the saliva collection tool 10, and a cap member 24 for sealing the container body 22. The container body 22 is pre-filled with a preservation solution such as a buffer for diluting and preserving saliva and sealed with the cap member 24 when provided. The preservation solution can be, for example, about 1-2 mL. The collection tool 10 is not housed in the container body 22 when provided (Figure 4).

A label 26 is attached on the periphery of the container body 22. The container body 22 is preferably formed to have a bottomed cylindrical shape. The label 26 is preferably printed with an ID barcode or a QR code (registered trademark), and more preferably has a blank name space for writing subject's information such as the subject's name and attributes.

The upper part of the cap member 24 preferably has a cap-side insertion opening 29 (dashed line) covered and closed with a seal 28. The seal 28 may preferably be a puncturable aluminum seal or a peelable seal. The length of the container body 22 can be, for example, 7-8 cm. Figure 1 shows the state where the collection tool 10 is entirely housed in the container 22 after the collection of saliva, etc.

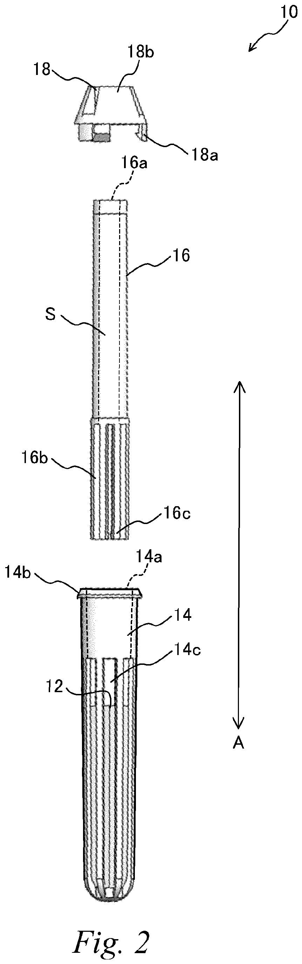

Next, the structure of the collection tool 10 will be described with reference to Figures 2-4. The collection tool 10 comprises a saliva absorbent (collecting body) 12 made of a material that can absorb saliva and is stretchable, a housing (support) 14 for housing (supporting) the saliva absorbent 12, a pressing body 16 for pressing the saliva absorbent 12, and a housing head 18 that is attached (secured) to the upper end of the housing 14.

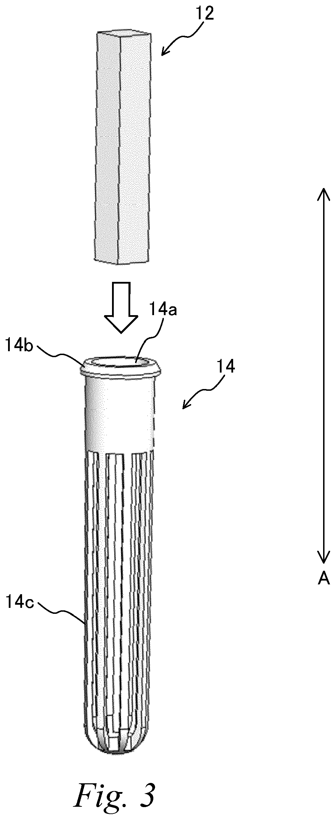

The shape of the saliva absorbent 12 may preferably be a rectangular prism, as shown in Figure 3. The shape of the saliva absorbent 12, however, is not limited to a rectangular prism, and can be a cylinder or a polygonal prism. The saliva absorbent 12 can be made of sponge, cotton or other fiber, or a soft porous material. The housing 14 can be made of a resin that is rigid enough not to deform during the process of pressing the saliva absorbent 12 described below. The housing 14 may preferably be an elongated basket body as shown in Figures 2 and 3. The outer diameter of the housing 14 is smaller than the inner diameter of the container body 22. The outer diameter of the housing 14 may preferably be 80%-90% of the inner diameter of the container body 22.

As shown in Figures 2 and 3, the housing 14 comprises an inlet 14a for receiving the saliva absorbent 12 and the pressing body 16 from its upper end, a rib (flange) 14b protruding outwardly from the periphery of the housing 14 around the inlet 14a, and one or a plurality of side openings (slits) 14c formed on the side of the housing 14. The side openings 14c open on the inner surface of the container body 22 in the state where the housing 14 is housed in the container body 22. Saliva and the preservation solution can enter inside the housing 14 through the side opening 14c. The housing 14 may preferably be obtained by molding a resin.

The pressing body 16 is tubular as a whole as shown in Figure 2, and its interior constitutes a suction space S for suctioning saliva and the preservation solution, as described below. The pressing body 16 comprises an upper end opening 16a, one or a plurality of side openings (slits) 16b formed on the lower side of the pressing body 15, and one or a plurality of elongated legs 16c forming the side openings 16b. The lower ends of the legs 16c make contact with the upper end of the saliva absorbent 12 when the saliva absorbent 12 is housed in the housing 14. The pressing body 16 may preferably be obtained by molding a resin.

As shown in Figure 2, the housing head 18 is flat and cylindrical or ring-shaped. The housing head 18 comprising a plurality of claws 18a annularly provided on the lower side of the housing head 18 and one or a plurality of protrusions 18b annularly provided on the upper side of the housing head 18. As shown in Figure 4, when the housing head 18 is attached to the housing 14, the claws 18a engage with the rib 14b to secure the housing head 18 to the housing 14. The one or the plurality of the protrusions 18b are angled toward the inside of the housing head 18. Since the tips of the one or the plurality of protrusions 18b make contact with the outer circumference of the pressure body 16 when the housing head 18 is secured to the housing 14 as shown in Figure 4, the pressing body 16 can be held slidably in the axial direction A with respect to the housing 14.

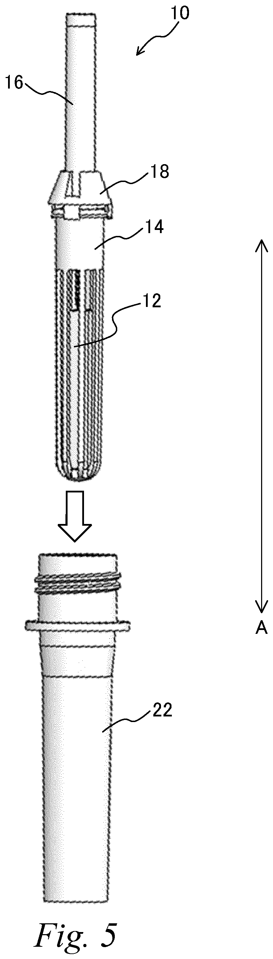

A method for collecting saliva using the collection kit 1 will be described with reference to Figures 5-7. The saliva collection kit 1 before saliva collection preferably comprises the collection tool 10 and the container body 22 (containing the preservation solution) sealed with the cap member 24, which can separately be enclosed in packages (not shown). The collector (e.g., the physician or the subject of collection) takes the collection tool 10 and the container body 22 out of the packages to collect saliva into the saliva absorbent 12 of the collection tool 10. Saliva can be absorbed (collected) into the saliva absorbent 12 for saliva collection preferably by holding the upper part of the housing 14 and/or the pressing body 16 of the collection tool 10 by hand and placing the lower part of the housing 14 housing the saliva absorbent 12 inside the mouth cavity, on the tongue, or under the tongue. The amount of saliva to be collected can be, for example, 1 mL or more.

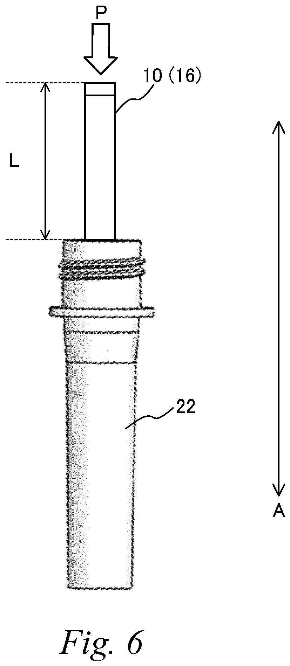

After the saliva absorbent 12 has absorbed saliva, the collection tool 10 is inserted into the container body 22 by the collector as shown in Figure 5. Once the saliva absorbent 12 is inserted, one end or the upper part (pressing body 16) of the collection tool 10 upwardly sticks out from the opening at the upper end of the container body 22 as shown in Figure 6. Since the saliva absorbent 12 is stretchable, the collector can make the saliva absorbent 12 compressed by pressing it in the direction of the arrow (P) shown in Figure 6. The saliva absorbent 12 may preferably be made of PVA sponge. The saliva absorbent 12 made of PVA sponge becomes stretchable as it absorbs saliva and the preservation solution. PVA sponge is made from polyvinyl alcohol (PVA) with continuous pores. PVA sponge is rigid in the dry state, but flexible and/or elastic in the hydrated state.

Figure 1 shows the state where the saliva absorbent 12 is sufficiently compressed. In the state shown in Figure 1, the collection tool 10 (pressing body 16) is housed entirely in the container body 22. The saliva absorbent 12 is preferably elastically deformable so that when the collector releases his/her hand from the collection tool 10, the pressure is released and the saliva absorbent 12 expands upward in the longitudinal direction A in the container body 22. By alternately repeating the pressing and releasing several times, the collected saliva is sufficiently dissolved in the preservation solution and stabilized.

As shown in Figure 6, when not pressed, the upper part of the collection tool 10 (pressing body 16) upwardly sticks out from the upper end of the container body 22 by a length L. The protrusion length L may be, for example, preferably at least 1 cm, and more preferably at least 2-3 cm, in order to provide sufficient pressure and release.

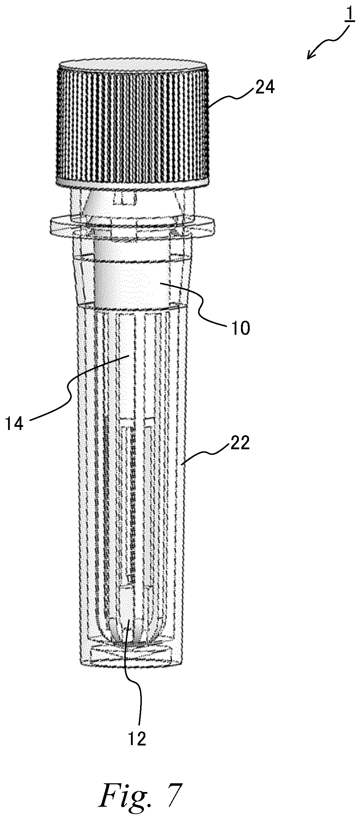

Figure 7 shows the collection kit 1 after the completion of saliva collection. The collection tool 10 and the container body 22 shown in Figure 1 are sealed with the cap member 24. Since the saliva absorbent 12 is compressible, it can be compressed by the screw-type cap member 24 when sealing the container body 22.

The collection kit 1 in the state shown in Figure 7 is transferred to a PCR testing device to analyze nucleic acids of a pathogen (virus, etc.) contained in the saliva. Preferably, a fully automated PCR testing system (geneLEAD series) provided by Precision System Science Co., Ltd. may be used for this analysis. This fully automated PCR testing system is capable of performing PCR test with the saliva collection kit 1 containing the collected saliva, without a manual intervention.

This fully automated PCR testing system comprises a dispenser 30 for suctioning the saliva-dissolved preservation solution from the collection tool 1. This fully automated PCR system preferably comprises a puncturing tool (not shown) for puncturing the seal 26 of the collection kit 1. Preferably, this fully automatic PCR testing system can automatically perform an extraction process for extracting the nucleic acids of a pathogen by performing pretreatment on the suctioned preservation solution containing saliva, an amplification process for amplifying the extracted nucleic acids by PCR method, and a detection process for detecting the amplified nucleic acids.

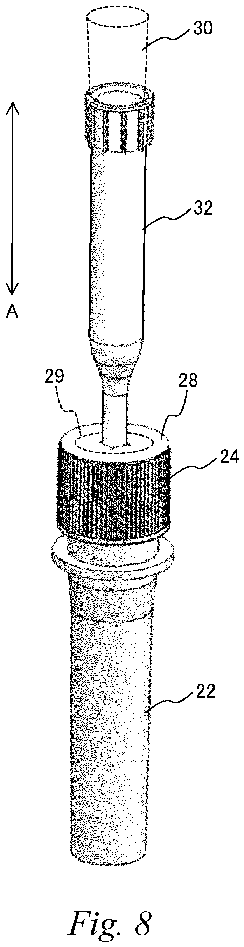

For the collection kit 1 containing the collected saliva, the fully automated PCR testing system punctures the seal 26 of the collection kit 1 with the puncturing tool and then inserts the lower end 32 of the dispenser 30 (dispensing tip 32 of dispenser 30) directly into the suction space S in the collection tool 10 housed in the container body 22 as shown in Figure 8, thereby directly suctioning the preservation solution with the dissolved saliva.

As shown in Figures 1 and 4, the pressing body 16 of the collection tool 10 has the upper end opening 16a and is formed to have a bottomed cylindrical shape as a whole, with the suction space S (Figure 2) and no protrusion or the like that obstructs the insertion of the lower end 32 of the dispenser 30. Therefore, when the preservation solution containing saliva, etc. is suctioned from the container body 22 with the dispenser 30, the lower end 32 of the dispenser 30 can be smoothly inserted through the punctured aluminum seal 26 of the cap member 24, the cap-side insertion opening 29, and the upper end opening 16a to the preservation solution containing saliva existing in the suction space S of the housing 14.

### [Second embodiment]

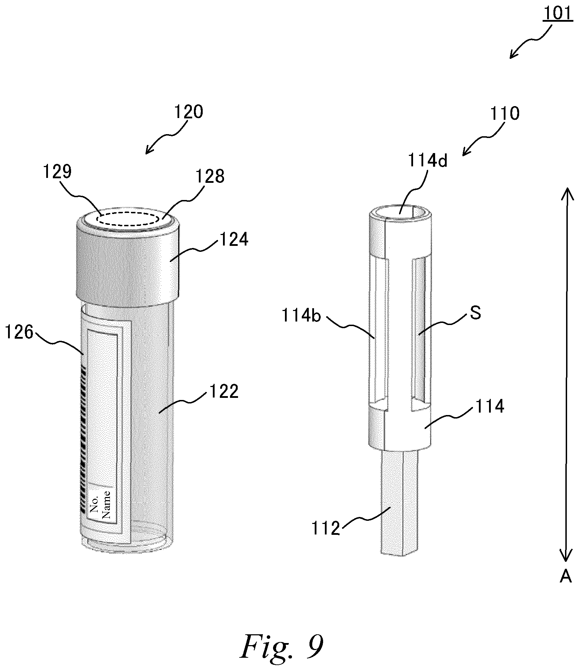

Overall configuration of a kit 101 for collecting a biological substance according to a second embodiment of the present invention will be described. As shown in Figure 9, the collection kit 101 comprises a biological substance (saliva) collection tool 110, and a container 120 for housing the collection tool 110 with the collected biological substance.

The collection tool 110 comprises a saliva absorbent 112 (collecting body) made of a stretchable material that can absorb liquid and a holder 114 (support) made of a nonstretchable material that is more rigid than the saliva absorbent 112 to support the saliva absorbent 112. The holder 114 is preferably cylindrical and accessible in the longitudinal (axial) direction A of the holder 114. The saliva absorbent 112 can be made of sponge, cotton or other fiber, or a soft porous material. The holder 114 may preferably be made of a resin that is rigid enough not to deform during the process of pressing the saliva absorbent described below. Preferably, the holder 114 is generally cylindrical in shape, and the outer diameter of the holder 114 is smaller than the inner diameter of the container body 122. The outer diameter of the holder 114 may preferably be 80%-90% of the inner diameter of the container body 122.

The container 120 comprises a bottomed cylindrical container body 122, a screw-type cap member 124 for sealing the container body 122, and a label 126 attached on the periphery of the container body 122. The container body 122 is preferably formed to have a bottomed cylindrical shape. The label 126 is preferably printed with an ID barcode or a QR code (registered trademark), and more preferably has a blank name space for writing subject's information such as the subject's name and attributes. The upper part of the cap member 124 preferably has a cap-side insertion opening 129 (dashed line) covered and closed with a seal 128. The seal 128 may preferably be a puncturable aluminum seal or a peelable seal. The length of the container body 122 can be, for example, 7-8 cm.

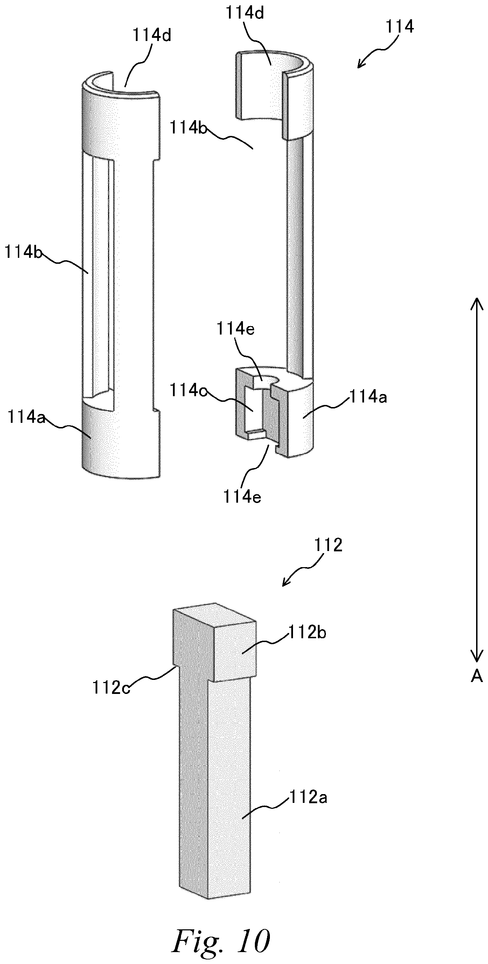

As shown in Figure 10, the holder 114 comprises a pair of arms 114a and 114a (a pair of supports or holding portions), one or a plurality of side openings 114b formed in the arms 114a and 114a, a holding section 114c having a dent formed at the lower end of each of the arms 114a and 114a to secure (clamp) the saliva absorbent 112, and a holder-side insertion opening 114d formed between the upper ends of the pair of arms 114a and 114a. The side openings 114b open on the inner surface of the container 122. The side openings 114b may preferably be formed at the junction between the pair of arms 114a and 114a or on the side of one arm 114a. The holding section 114c preferably has holes 114e at both of the upper and lower ends to allow communication of a preservation solution B. The holder 114 may preferably be obtained by molding a resin.

As shown in Figure 10, the saliva absorbent 112 comprises an absorbing portion 112a (collecting portion) extending in the longitudinal (axial) direction A of the saliva absorbent 12 and a catch portion 112b connected to the upper end of the absorbing portion 112a. Although the absorbing portion 112a is a rectangular prism in Figure 10, it can have any columnar shape including a polygonal prism, a cylinder, or a sphere, etc. The length of the absorbing portion 112a in the longitudinal direction A may preferably be 3-7 cm, more preferably about 5 cm. The catch portion 112b protrudes from the absorbing portion 112a in a direction substantially perpendicular to the longitudinal (axial) direction A, thereby forming a projecting portion 112c.

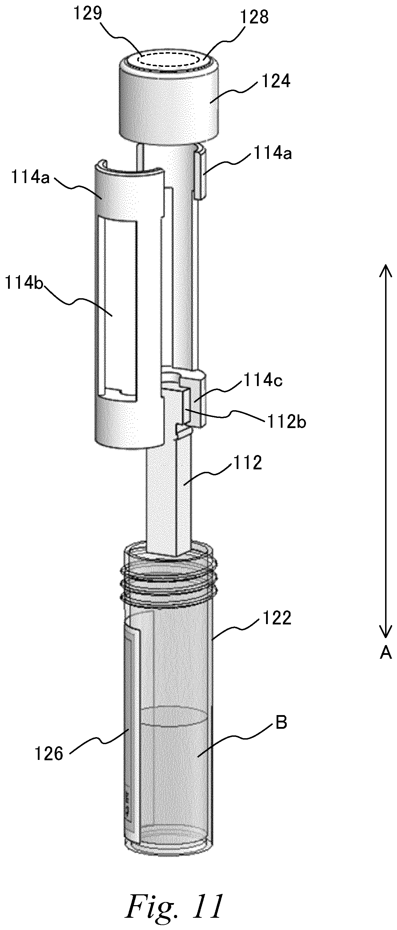

Figure 11 shows the assembled state of the collection kit 101. The container body 122 is pre-filled with a preservation solution B such as a buffer for diluting and preserving a body fluid such as saliva. The arms 114a and 114a are integrated into a single unit in the state where the catch portion 112b of the saliva absorbent 112 is held by the securing portion (holding section) 114c (Figure 11) of the holder 114. Integration of the pair of arms 114a and 114a into a single unit is realized by means of fitting or adhesion. Thus, the saliva absorbent 112 is secured to the holder 114. In this secured state, the saliva absorbent 112 and the holder 114 are housed in the container body 122 filled with the preservation solution B, and the container body 122 is sealed with the cap member 124. The preservation solution B can be, for example, about 1-2 mL.

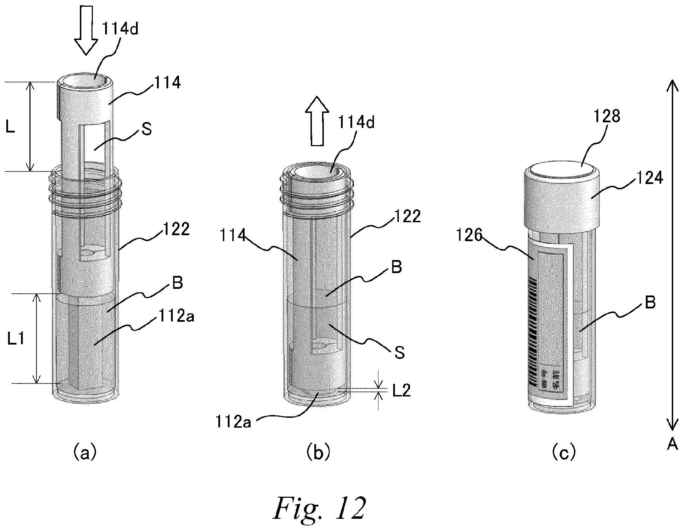

With reference to Figure 12, a method for collecting saliva using the collection kit 101 will be described. Before saliva collection, the collection kit 101 preferably has the collection tool 110 and the sealed container 120 (containing preservation solution B) separately enclosed in packages (not shown). The collector (e.g., the physician or the subject of collection) takes the collection tool 110 and the container 120 out of the packages and collects saliva into the saliva absorbent 112 of the collection tool 110. Saliva can be absorbed (collected) into the saliva absorbent 112 for saliva collection preferably by holding the holder 114 of the collection tool 110 by hand and placing the saliva absorbent 112 inside the mouth cavity, on the tongue, or under the tongue. The amount of saliva to be collected can be, for example, 1 mL or more.

After the saliva absorbent 112 has absorbed saliva, the collection tool 110 is inserted into the container body 122 from the saliva absorbent 112 side by the collector and the stretchable saliva absorbent 112 is pressed in the direction indicated by the arrow as shown in Figure 12 (a). Next, when the collector releases his/her hand from the collection tool 110 in the state where the saliva absorbent 112 is compressed as shown in Figure 12(b), the pressure is released and the saliva absorbent 112 expands in the longitudinal direction A in the container body 122 as shown in Figure 12(a). By alternately repeating the pressing and releasing several times, the collected saliva is sufficiently dissolved into the preservation solution and stabilized.

As shown in Figure 12(a), when not pressed, the upper part of the support 114 upwardly sticks out from the upper end of the container body 122 by a length L. As shown in Figure 12(a), when the container is sufficiently pressed, the upper side of the support 114 is housed within (at the same height as or at a lower level than) the upper end of the container body 122. The protrusion length L can be, for example, 80-90% of the longitudinal length of the unpressed absorbing portion 112a. Alternatively, the protrusion length L can be, for example, the longitudinal length L1 of the unpressed absorbing portion 112a minus the longitudinal length L2 of the pressed absorbing portion 112a (Figure 12(b)). The protrusion length L may be, for example, preferably at least 1 cm, and more preferably at least 2-3 cm, in order to provide sufficient pressure and release.

Figure 12(c) shows the state where the collecting body 110 and the container body 122 in the state shown in Figure 12(b) are sealed with the cap member 124. In the state shown in Figures 12(b) and 12(c), the liquid level of the preservation solution B is located in the suction space S formed inside the support 114. Since the absorbing portion 112a is compressible, it can be compressed and sealed with the screw-type cap member 124.

The collection kit 101 in the state shown in Figure 12(c) is transferred to a PCR testing device to analyze nucleic acids of a pathogen (virus, etc.) contained in the saliva. Preferably, a fully automated PCR testing system (geneLEAD series) provided by Precision System Science Co., Ltd. may be used for this analysis. This fully automated PCR testing system is capable of performing PCR test with the saliva collection kit 101 containing the collected saliva, without a manual intervention.

This fully automated PCR testing system comprises a dispenser 30 for suctioning the saliva-dissolved preservation solution B from the collection tool 101. This fully automated PCR system preferably comprises a puncturing tool (not shown) for puncturing the seal 128 of the collection kit 101. Preferably, this fully automatic PCR testing system can automatically perform an extraction process for extracting the nucleic acids of a pathogen by performing pretreatment on the suctioned preservation solution containing saliva, an amplification process for amplifying the extracted nucleic acids by PCR method, and a detection process for detecting the amplified nucleic acids.

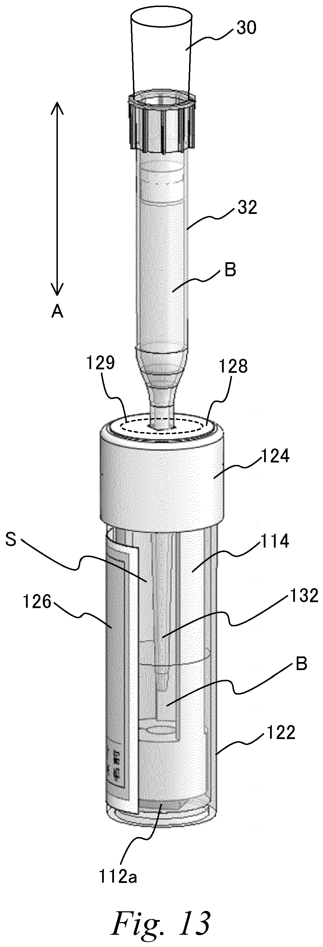

For the collection kit 101 containing the collected saliva, the fully automated PCR testing system punctures the seal 128 of the collection kit 101 with the puncturing tool and then insert the lower end 32 of the dispenser 30 (dispensing tip 32 of dispenser 30) directly into the suction space S in the collection kit 101 as shown in Figure 13, thereby directly suctioning the preservation solution with the dissolved saliva.

As shown in Figure 13, the collecting body 110 has a holder-side insertion opening 114d and is formed to have a cylindrical shape with a suction space S and no protrusion or the like that obstructs the insertion of the lower end 32 of the dispenser 30. Therefore, when the preservation solution B containing saliva is suctioned from the container body 122 with the dispenser 30, the lower end 32 of the dispenser 30 can be smoothly inserted through the punctured aluminum seal 128 and the cap-side insertion opening 129 of the cap member 124 to the preservation solution B containing saliva existing in the suction space S of the support 114.

The preservation solutions such as buffers used in the first and second embodiments may contain each of the following additives as needed for collecting saliva. The additives may be any one or more of the following: a coagulation inhibitor for inhibiting coagulation of mucin contained in saliva; a diluent for diluting mucin to inhibit coagulation; an inactivator for inactivating an enzyme contained in saliva; a stabilizer for inhibiting degradation of RNAs of a virus in saliva to stabilize them; and a protease or protein denaturant for breaking down or denaturing a protein (envelope or capsid) of a virus in saliva.

- 1: Collection kit
- 10: Collection tool
- 12: Saliva absorbent (collection tool)
- 14: Housing (retainer)
- 16: Pressing body
- 18: Housing head
- 22: Container body
- 24: Cap member
- 26: Label
- 28: Seal
- 29: Cap-side insertion opening
- 30: Dispenser
- A: Longitudinal (axial) direction
- P: Direction of pressure
- 101: Collection kit
- 110: Collection tool
- 112: Saliva absorbent (collecting body)
- 114: Holder (support)
- 114a: Arm section
- 114b: Side opening
- 114c: Holding section
- 114d: Holder-side insertion opening
- 120: Container
- 122: Container body
- 124: Cap member
- 126: Label
- 128: Seal
- 129: Cap-side insertion opening
- B: Preservation solution
- S: Suction space

## Claims

1. A kit for collecting a biological substance, the kit comprising:
a collection tool for collecting the biological substance;
a container body for housing the collection tool and a preservation solution for preserving the biological substance; and
a cap member for sealing the container body,
wherein the collection tool comprises a collecting body for collecting the biological substance, a housing for housing the collecting body, and a suction space for suctioning the biological substance and the preservation solution from the outside of the container body.

2. The kit for collecting a biological substance according to Claim 1, wherein the cap member comprises a cap-side insertion opening connecting to the suction space, and a seal for sealing the cap-side insertion opening.

3. The kit for collecting a biological substance according to Claim 2, wherein the seal is puncturable from the outside.

4. The kit for collecting a biological substance according to any one of Claims 1 to 3,
wherein the collecting body is formed of a stretchable material that can absorb the biological substance and the preservation solution; and
wherein in an uncompressed state where the collecting body placed in the container body is not compressed, one end of the collection tool sticks out from the container body.

5. The kit for collecting a biological substance according to Claim 4, wherein, in a compressed state where the collecting body placed in the container body is compressed, said one end of the collection tool is housed in the container body.

6. The kit for collecting a biological substance according to Claim 5, wherein the container body in the compressed state is sealed with the cap member.

7. The kit for collecting a biological substance according to any one of Claims 1 to 6, wherein the housing has one or a plurality of openings.

8. The kit for collecting a biological substance according to any one of Claims 1 to 7, wherein the housing is an elongated basket body.

9. The kit for collecting a biological substance according to any one of Claims 1 to 8, wherein the collection tool comprises a pressing body that presses the collecting body toward the housing.

10. The kit for collecting a biological substance according to Claim 9, wherein the pressing body forms the suction space.

11. The kit for collecting a biological substance according to either one of Claims 10 and 11, wherein the pressing body is tubular.

12. The kit for collecting a biological substance according to any one of Claims 9 to 11, wherein the collection tool comprises a housing head that holds the pressing body in a slidable manner with respect to the collecting body.

13. The kit for collecting a biological substance according to Claim 12, wherein the housing head is secured to the housing.

14. The kit for collecting a biological substance according to either one of Claims 12 and 13, wherein the housing head comprises one or a plurality of protruding portions that make contact with the pressing body.

15. A kit for collecting a biological substance, the kit comprising:
a collection tool for collecting the biological substance;
a container body for housing the collection tool and a preservation solution for preserving the biological substance; and
a cap member for sealing the container body,
wherein the collection tool comprises a collecting body for collecting the biological substance, and a support for holding the collecting body and forming a suction space for suctioning the biological substance and the preservation solution from the outside of the container body.

16. The kit for collecting a biological substance according to Claim 15, wherein the cap member comprises a cap-side insertion opening connecting to the suction space, and a seal for sealing the cap-side insertion opening.

17. The kit for collecting a biological substance according to Claim 16, wherein the seal is puncturable from the outside.

18. The kit for collecting a biological substance according to any one of Claims 15 to 17, wherein:
the collecting body is formed of a stretchable material that can absorb liquid; and
in an uncompressed state where the collecting body placed in the container body is not compressed, one end of the support sticks out from the container body.

19. The kit for collecting a biological substance according to Claim 18, wherein, in a compressed state where the collecting body placed in the container body is compressed, said one end of the support is housed in the container body.

20. The kit for collecting a biological substance according to Claim 19, wherein the container body in the compressed state is sealed with the cap member.

21. The kit for collecting a biological substance according to Claim 20, wherein the preservation solution is contained in the container body in a liquid amount such that, in the compressed state, the liquid level of the preservation solution is located in the suction space.

22. The kit for collecting a biological substance according to any one of Claims 15 to 21, wherein the support comprises a support-side insertion opening for suctioning the biological substance and the preservation solution from the outside.

23. The kit for collecting a biological substance according to any one of Claims 15 to 22, wherein the support comprises one or a plurality of side openings opening on the inner surface of the container body.

24. The kit for collecting a biological substance according to any one of Claims 15 to 23, wherein the support comprises a holding section for holding the collecting body.

25. The kit for collecting a biological substance according to Claim 24, wherein the holding section comprises holes at its upper and lower ends, each of which allowing communication of the preservation solution and the biological substance.

26. The kit for collecting a biological substance according to any one of Claims 1 to 25, wherein the container body comprises a label, and the label has at least one of a bar code, a QR code (registered trademark), and a blank name space on which information of the subject can be written.

27. The kit for collecting a biological substance according to any one of Claims 1 to 26, wherein the biological substance is saliva.

28. A method for collecting a biological substance using the kit for collecting a biological substance according to any one of Claims 1 to 27, the method comprising the steps of:
collecting the biological substance into the collection tool;
housing the collection tool in the container body;
compressing the collecting body by pressing one end of the pressing body to put the collecting body into the compressed state;
expanding the collecting body by releasing the pressure at one end of the pressing body to put the collecting body into the uncompressed state; and
sealing the container body with the cap member, with the collecting body in the compressed state.

29. The method for collecting a biological substance according to Claim 28, wherein the step of compressing the collection tool and the step of expanding the collection tool are repeated alternately.

30. A testing device for testing a biological substance collected using the kit for collecting a biological substance according to any one of Claims 1 to 27, the device comprising a dispenser for suctioning the biological substance and the preservation solution from the suction space.

31. A testing device for testing a biological substance collected using the kit for collecting a biological substance according to either one of Claims 3 and 17, the device comprising:
a puncturing tool for puncturing the puncturable seal; and
a dispenser that is inserted into the suction space via the cap-side insertion opening to suction the biological substance and the preservation solution from the suction space.

32. The testing device according to either one of Claims 30 and 31, wherein the testing device extracts nucleic acids from the biological substance, amplifies the extracted nucleic acids, and tests the amplified nucleic acids.
